# EUROPEAN PATENT APPLICATION

(11) **EP 0 586 824 A1**
(43) Date of publication of application: **16.03.1994**
(21) Application number: 93110932.6
(22) Date of filing: 08.07.1993
(51) Int. Cl.: A61B 17/58

(54) **Internal prosthesis for synthesis of the proximal femur region**

(30) Priority: 13.07.1992 IT BO920277
(71) Applicant: Battaglia, Luigi, I-47100 Forli' (IT)
(72) Inventor: Battaglia, Luigi, I-47100 Forli' (IT)
(74) Representative: Modiano, Guido, Dr.-Ing.

(57) **Abstract**

Internal prosthesis (1) for synthesis of the proximal femur region (f1, f2, f3), comprising an intramedullary rod (2) provided with an upper half-part (3), which forms an obtuse angle with respect to the lower half-part, and with a lower half-part (4), which is affected by at least one thick longitudinal through notch (5); the upper half-part is crossed by an oblique hole (14) along which the body (15) of a nail (16) is slideably fitted; the portion (17) of the nail which is meant to couple to the bone is affected by radial fins (18) for preventing rotation, and between the nail body and the hole of the rod there are means (21, 22) suitable to prevent their mutual rotation.

## Description

The present invention relates to an internal prosthesis for synthesis of the proximal femur region, i.e. a prosthesis which is installed following a fracture proximate to the head of the femur during an operation and is meant to act as a mechanical support while waiting for the consolidation and restoration of the load-bearing capacity of the bone.

The invention solves a considerable number of cases and in particular transcervical fractures, basal neck fractures, pertrochanteric fractures and subtrochanteric fractures.

Internal prostheses constituted by a rod which is meant to be inserted in the medullary canal of the femur and to which a screw can be rigidly fixed, said screw being then screwed into the head of the femur, are known for femur synthesis; said known devices have numerous shortcomings and a significant number of drawbacks.

First of all, the rod is massive and has an inadequate shape, with an excessive angle between its proximal part and its distal part, so much that in some cases, during insertion or use, it can break through the walls of the femur with its lower end.

Secondly, a possibility of mutual rotation remains between the screw and the head of the femur; this possibility must be eliminated by means of a special screw which is inserted from above within the structure of the rod, until the head screw is compressed, and by means of other transverse screws which cannot be placed quickly.

A principal object of the present invention is to obviate the above problems of known devices, i.e. to provide an internal prosthesis for synthesis of the proximal femur region by means of which the intramedullary rod causes no problems to the femur, mutual rotations among the parts are avoided by virtue of means which do not entail increases in the duration of the operation, and the natural contraction of bone volume during fracture consolidation is allowed.

Another object of the present invention is to provide an internal prosthesis which can be installed with a relatively scarcely traumatic operation which can be carried out on extremely small surgical areas, both for placement and for possible final removal once the fracture has healed.

Another object of the present invention is to provide an internal prosthesis for synthesis of the proximal femur region, the structure whereof is simple, relatively easy to produce in practice, safe in use, and effective in operation as well as relatively modest in cost.

With these and other objects in view, there is provided, according to the present invention, an internal prosthesis for synthesis of the proximal femur region, characterized in that it comprises an intramedullary rod provided with an upper half-part, which forms an obtuse angle with respect to the lower half-part, and with a lower half-part, which is affected by at least one thick longitudinal through notch; the upper half-part is crossed by an oblique hole along which the body of a nail is slideably fitted; the portion of said nail which is meant to couple to the bone is affected by radial fins for preventing rotation, and between the nail body and the hole of the rod there are means suitable to prevent their mutual rotation.

The particular characteristics of the present invention will become apparent and evident from the following detailed description of a preferred but not exclusive embodiment thereof illustrated only by way of non-limitative example in the accompanying drawings, wherein:

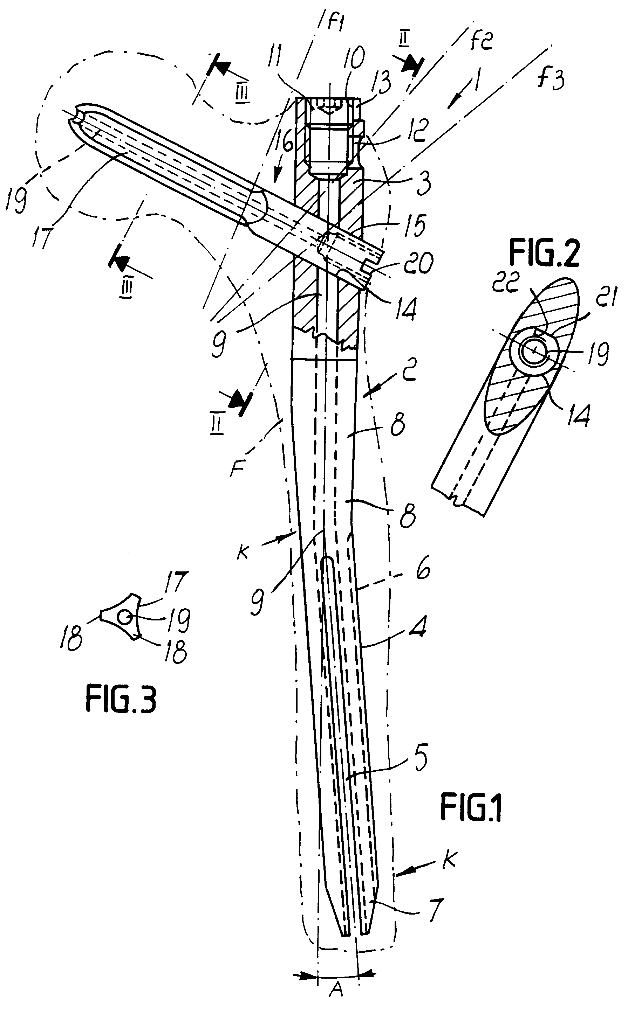

figure 1 is a partially sectional side view of an internal prosthesis according to the invention;
figure 2 is a sectional view, taken along the plane II-II of figure 1;
figure 3 is a sectional view, taken along the plane III-III of figure 1.

With particular reference to the above figures, the reference numeral 1 generally designates the internal prosthesis for the synthesis of the proximal region of the femur F, according to the invention; said femur may be fractured, by way of example, at the proximal region approximately along the fracture lines f1, f2, and f3.

The internal prosthesis 1 comprises an intramedullary rod 2 constituted by an upper half-part 3 which forms an angle A, preferably of six degrees, with respect to the lower half-part 4; the purpose of this angle is to produce a rather forced contact between two oppositely located points of the rod and the walls of the femoral medullary canal, in order to prevent the rotation of the rod within the femoral structure: these points of forced contact have been approximately designated by the letter K in the figure.

The lower half-part 4 is affected by at least one thick longitudinal through notch 5 and has a circular crosssection which is crossed by a few longitudinal grooves 6 (three are provided in the particular case) which are suitable to contrast its rotation; the end 7 of the lower half-part 4 has a converging frustum-like shape.

The upper half-part 3 connects to the lower half-part 4, which has a smaller or at the most equal diameter, with a possible frustum-shaped median portion 8; the different diameters of the lower half-part allow adaptation to all sizes of the human femur; the rod is longitudinally crossed by a through hole 9 for a known insertion guiding wire.

The top of the rod has an axial hole 10 which is bigger than 9 and is threaded for the screw coupling of an actuation handle (instrument) or, once the instrument has been removed, of a closure plug 11 which prevents the forming of bone formations in the hole; such formations would then have to be removed in case of extraction of the prosthesis; in order to remove the rod, a lateral coupling slot 12 is provided proximate to the edge of the hole 10; at the edge of 10 there is also a notch 13 for fixing the instrument.

The upper half-part 3 is crossed by an oblique hole 14 along which the body 15 of a nail 16 is slideably fitted; the portion 17 of said nail is meant to couple to the bone and is affected by radial fins 18 for preventing rotation (in the particular case shown in the figure, there are three fins and the nail is of the known type termed triple-flange nail): the angle formed by the hole 14 with respect to the axis of the rod can assume different values, for example 125, 130 or 135 degrees, in order to duplicate the natural (cervical-diaphyseal) angle of inclination of the human femur.

The nail 16 is longitudinally crossed by a hole 19 for the passage of an insertion guiding wire; the end of the body 15 has a threaded hole 19 and four notches 20 on the inlet for the fitting instrument.

Between the nail body 15 and the hole 14 of the rod there are means suitable to prevent their mutual rotation; said means are constituted by a snug-fit sliding coupling between the cylindrical body 15 of the nail 16, which is provided, for this purpose, with a flattened surface 21, and a complementary flattened portion 22 of the hole 14 of the rod.

The thick notch 5 and the frustum-shaped end 7 of the lower half-part of the rod give said rod a certain deformability and appropriate shape to prevent its lower end from damaging the bone; the finned shape of the nail couples it to the head of the femur so as to prevent rotation without requiring further mechanical action; the possibility of longitudinal sliding, but not of rotation, of the nail with respect to the rod allows the longitudinal sliding movements due to natural bone volume contraction during healing; in addition to this, the operation required to apply the internal prosthesis according to the invention is scarcely invasive, since two small accesses are sufficient: one in alignment with the half-part 3 of the rod and the other in alignment with the nail 16.

It has thus been observed that the invention achieves the intended objects.

The invention thus conceived is susceptible to numerous modifications and variations, all of which are within the scope of the inventive concept.

All the details may furthermore be replaced with other technically equivalent ones.

In practice, the materials employed, as well as the shapes and dimensions, may be any according to the requirements without thereby abandoning the protective scope of the following claims.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Internal prosthesis (1) for synthesis of the proximal femur region, characterized in that it comprises an intramedullary rod (2) provided with an upper half-part (3), which forms an obtuse angle with respect to the lower half-part, and with a lower half-part (4), which is affected by at least one thick longitudinal through notch (5); the upper half-part is crossed by an oblique hole (14) along which the body (15) of a nail (16) is slideably fitted; the portion (17) of said nail which is meant to couple to the bone is affected by radial fins (18) for preventing rotation, and between the nail body (15) and the hole (14) of the rod (2) there are means (21, 22) suitable to prevent their mutual rotation.

2. Internal prosthesis according to claim 1, characterized in that the lower end (7) of said rod is convergent and frustum-shaped.

3. Internal prosthesis according to the preceding claims, characterized in that said rod (2) is longitudinally crossed by a through hole (9) for an insertion guiding wire.

4. Internal prosthesis according to the preceding claims, characterized in that the top of the rod has an internally threaded axial hole (10) for the screw coupling of an actuation handle (instrument) or of a closure plug (11).

5. Internal prosthesis according to the preceding claims, characterized in that the lower half-part (4) of the rod is provided with longitudinal grooves (6).

6. Internal prosthesis according to the preceding claims, characterized in that said means suitable to prevent mutual rotation between the body of the nail and the rod are constituted by a snug-fit sliding coupling between the cylindrical body of the nail, which has a flattened surface (21), and a complementary flattened portion (22) of a circular hole of the rod.

7. Internal prosthesis according to the preceding claims, characterized in that said nail is longitudinally crossed by a through hole (19) for an insertion guiding wire.
